(19) Europäisches Patentamt
European Patent Office
Office européen des brevets

(11) **EP 2 285 871 B1**

(12) **EUROPÄISCHE PATENTSCHRIFT**

(45) Veröffentlichungstag und Bekanntmachung des Hinweises auf die Patenterteilung:
**14.12.2011 Patentblatt 2011/50**

(21) Anmeldenummer: **09753405.1**

(22) Anmeldetag: **27.05.2009**

(51) Int Cl.:
*A61F 2/46* *(2006.01)*    *A61L 27/16* *(2006.01)*
*C08L 29/04* *(2006.01)*    *A61L 27/52* *(2006.01)*
*C08J 3/075* *(2006.01)*    *A61F 2/44* *(2006.01)*

(86) Internationale Anmeldenummer:
**PCT/CH2009/000178**

(87) Internationale Veröffentlichungsnummer:
**WO 2009/143646 (03.12.2009 Gazette 2009/49)**

(54) **NUCLEUS ERSATZ BASIEREND AUF INJIZIERBAREN PVA GELEN**

NUCLEUS REPLACEMENT BASED ON INJECTABLE PVA GELS

SUBSTITUT DE NOYAU À BASE DE GELS DE PVA INJECTABLES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **27.05.2008 PCT/CH2008/000236**

(43) Veröffentlichungstag der Anmeldung:
**23.02.2011 Patentblatt 2011/08**

(73) Patentinhaber: **InnoGEL AG**
**6331 Hünenberg (CH)**

(72) Erfinder:
• **MÜLLER, Rolf**
**CH-8055 Zürich (CH)**
• **INNEREBNER, Federico**
**CH-8049 Zürich (CH)**
• **STEFFEN, Thomas**
**CH-3076 Worb (CH)**
• **BOOS, Norbert**
**CH-8342 Wernetshausen (CH)**

(74) Vertreter: **Becker Kurig Straus**
**Patentanwälte**
**Bavariastrasse 7**
**80336 München (DE)**

(56) Entgegenhaltungen:
WO-A-92/10982          WO-A-2005/017000
WO-A-2006/021122    WO-A-2007/050744
WO-A-2008/031235    US-A- 3 933 587
US-B1- 7 214 245

• **NOGUCHI ET AL: "Poly(Vinyl Alcohol) Hydrogel As an Artificial Articular Cartilage: Evaluation of Biocompatibility" JOURNAL OF APPLIED BIOMATERIALS, JOHN WILEY & SONS, INC., NEW YORK, NY, US, Bd. 2, 1. Januar 1991 (1991-01-01), Seiten 101-107, XP002431036 ISSN: 1045-4861 in der Anmeldung erwähnt**
• **R.A. BADER AND W.E. ROCHEFORT: "Rheological characterization of photopolymerized poly(vinyl alcohol) hydrogels for potential use in nucleus pulposus replacement" JOURNAL OF BIOMEDICAL MATERIALS RESEARCH PART A, Bd. 86a, Nr. 2, 1. November 2007 (2007-11-01), Seiten 494-501, XP002518823 in der Anmeldung erwähnt**
• **J. THOMAS ET AL.: "Novel associated hydrogels for nucleus pulposus replacement" JOURNAL OF BIOMEDICAL RESEARCH PART A, Bd. 67A, Nr. 4, 2003, Seiten 1329-1337, XP002518824 in der Anmeldung erwähnt**

EP 2 285 871 B1

## Beschreibung

Die Erfindung betrifft neue für Nucleus (Gelkörper der Bandscheiben) Ersatz geeignete in situ gelierende Polyvinylalkohol (PVA) Gele und ein Verfahren und eine Vorrichtung zu deren Applikation, ihre Verwendung und einen Behälter, welcher ein erfindungsgemäßes PVA Gel enthält.

Stand der Technik

T. Noguchi, T. Yamamuro et al. haben in Journal of Applied Biomaterials Bd. 2, 1991, Seiten 101 - 107 gezeigt, dass Hydrogele auf Basis von Polyvinylalkohol (PVA) mit der Konsistenz von biologischem Gewebe und Knorpel hergestellt werden können und im lebenden Organismus eine herausragende Stabilität und Biokompatibilität aufweisen, welche einerseits im hohen Wassergehalt dieser Gele und andererseits im Makromolekül selbst begründet ist, das vom Organismus infolge der zahlreichen Hydroxyl Gruppen ähnlich wie Wasser wahrgenommen wird. Daher sind PVA Gele für Anwendungen im lebenden Organismus geradezu prädestiniert.

R. A. Bader und W. E. Rochefort geben in Journal of Biochemical Materials Research Part A, Bd. 86a Nr. 2, 2007, Seiten 494 - 501 ein Beispiel für die Verwendung eines PVA Hydrogels als künstlicher Gelenkknorpel. Das PVA Gel wird hierbei allerdings strahlenvernetzt.

Es ist aus der WO 2005/017000 A1 bekannt, PVA Gele als Nucleus Ersatz einzusetzen. Diese benötigen jedoch zur Aushärtung den Zusatz eines Gelierungsmittels, wie beispielsweise eines Salzes oder anderer problematischer Chemikalien. In ähnlicher Weise beschreibt die WO 2007/050744 ein PVA Gel als Nucleus Ersatz, dessen Aushärtung durch Zusatz eines Vernetzungsmittels eingeleitet wird.

Die US 7,214,245 B1 offenbart ein Hydrogel auf Basis eines Blends aus PVA und PVP (Polyvinylpyrollidon) zum Einsatz als Nucleus Ersatz. J. Thomas et al. untersuchen in Journal of Biomedical Research Part A Bd. 67a Nr. 4, 2003, Seiten 1329 - 1337 solche Hydrogele auf Basis von PVA und PVP. Sie kommen zu dem Ergebnis, dass diese Gele dazu neigen PVP Ketten abzugeben, was eine starke Quellung und schlechte mechanische Eigenschaften zur Folge hat.

Die WO 92/10982 A1 beschreibt einen Nucleus Ersatz aus einem PVA Gel. Dieses wird außerhalb des Körpers in einem Gemisch aus Wasser und einem organischen Lösungsmittel angesetzt und in einer Forma ausgehärtet. Anschließend muss das organische Lösungsmittel durch Wasser ausgetauscht werden, bevor der Nucleus Ersatz in den Körper eingesetzt werden kann.

Beim Nucleus Ersatz ist die Viskosität der zu injizierenden Masse ist ein entscheidender Parameter um eine gute Füllung einer ausgeräumten Bandscheibe zu erhalten. Bei genügend hoher Viskosität kann eine gute Füllung erreicht werden, indem bei der Injektion ein Druck innerhalb der Bandscheibe aufgebaut werden kann. Dabei kann sogar mindestens teilweise die ursprüngliche Höhe der Bandscheibe wiederhergestellt werden. Ist die Injektion beendet und wird die Injektionsnadel zurückgezogen, so fliesst die deponierte Masse infolge der hohen Viskosität nicht mehr zurück. Bei zu tiefen Viskositäten kann der genannte Druck nicht aufgebaut werden und kann die deponierte Masse aus der Kavität hinaus fliessen, bevor sie genügend geliert hat, um das Fliessen zu verhindern. Ein solches in situ, d. h. am Einsatzort, gelierende PVA Gel mit zu tiefer Viskosität ist aus der WO 2006/021122 A2 bekannt.

Das vorherrschende Versagenskriterium bei bisherigen Lösungen im Bereich Nucleus Replacement ist die Extrusion, d.h. das mindestens teilweise Austreten des Gels aus dem Nucleus Raum.

Es ist daher Aufgabe der vorliegenden Erfindung, ein verbessertes in situ gelierendes PVA Gel bereitzustellen, welches hinsichtlich seines Verarbeitungsverhaltens, insbesondere betreffend der Viskosität und der Geliergeschwindigkeit, sowie betreffend seine Endeigenschaften, insbesondere E-Modul und Extrusionsverhalten, für die Anwendung als injizierbares Gel zum Ersatz von Nucleus Material mittels minimal invasiver Methode massgeschneidert ist.

Kurze Beschreibung der Erfindung

Diese Aufgabe wird erfindungsgemäß durch ein in situ gelierendes PVA Gel gelöst, welches PVA und Wasser als Quellungsmittel aufweist. Der Wassergehalt $W$ des PVA Gels liegt im Bereich 52 - 64 Gew.%. Die obere Grenze für das Gewichtsmittel des Polymerisationsgrades $P_w$ des PVA ist durch die Beziehung $P_w = W \cdot 150 - 5500$ gegeben, und die untere Grenze für das Gewichtsmittel des Polymerisationsgrades $P_w$ des PVA ist durch die Beziehung $P_w = W \cdot 90 - 3800$ gegeben. Die Viskosität des geschmolzenen Gels liegt bei einer Temperatur im Bereich 30 bis 100°C im Bereich 60 - 2000 Pas, wobei im Sinne der Erfindung unter Viskosität die dynamische Viskosität zu verstehen ist.

Mit abnehmendem Wassergehalt nimmt die Viskosität wie auch die Gelierungsgeschwindigkeit zu. Da übliche PVA-Wasser Mischungen vergleichsweise langsam gelieren, ist eine Einflussnahme auf diesen Parameter von grosser Bedeutung. Denn eine behandelte Bandscheibe sollte spätestens 1h nach der Operation mindestens minimal belastet werden können. Die untere Grenze des Wassergehalt ergibt sich einerseits durch die Viskosität, die bei zu tiefen Wassergehalten zu hoch wird, die obere Grenze des Wassergehalts ergibt sich aus der Viskosität die zu tief wird und aus der Gelierungsgeschwindigkeit, die zu tief wird. Die untere Grenze für den Wassergehalt des PVA Gels in Gew.% liegt daher bevorzugt bei 54, besonders bevorzugt 55, ganz besonders bevorzugt 56. Die obere Grenze für den Wassergehalt des PVA Gels in Gew.% liegt bevorzugt bei 62, besonders bevorzugt 61, ganz besonders bevorzugt 60. Der Wassergehalt ergibt sich aus dem Gewicht des Wassers dividiert durch das Gewicht des PVA Gels. Enthält das Gel einen in einer separaten Phase vorliegenden Füllstoff, so wird dieser nicht zum Gel gerechnet.

Das Gewichtsmittel des Polymerisationsgrades $P_w$ des PVA ergibt sich als Summe $w_i$ mal $P_{wi}$, wobei $w_i$ die Gewichtsfraktion der Spezies $PVA_i$ mit dem Gewichtsmittel des Polymerisationsgrades $P_{wi}$ ist:

$$P_{wi} = \Sigma \, (w_i \cdot P_{wi})$$

Besteht z.B. das PVA aus einem Typ von PVA, so ist $P_w$ der $P_w$ dieses PVA Typs. Besteht das PVA aus zwei Typen von PVA, wobei PVA1 40 Gew.% und PVA2 60 Gew.% des PVA ausmacht, so ist $P_w$ = 0,4 x $P_{w1}$ + 0,5 x $P_{w2}$.

In einer bevorzugten Ausführungsform liegt die untere Grenze von $P_w$ bei einem Wassergehalt W von 50 Gew% bei 750, während sie bei einem Wassergehalt W von 70 Gew% bei 2600 liegt. Bei Wassergehalten zwischen 50 und 70% gelten linear interpolierte Werte, d. h. $P_w$ = W·92,5 - 3875. In einer besonders bevorzugten Ausführungsform liegt die untere Grenze von $P_w$ bei einem Wassergehalt W von 50 Gew% bei 800, während sie bei einem Wassergehalt von 70% bei 2700 liegt, d. h. $P_w$ = W·95 - 3950. In einer besonders bevorzugten Ausführungsform liegt die untere Grenze von $P_w$ bei einem Wassergehalt W von 50 Gew% bei 900, während sie bei einem Wassergehalt von 70% bei 2800 liegt, d. h. $P_w$ = W·95 - 3850.

In einer bevorzugten Ausführungsform liegt die untere Grenze von $P_w$ bei einem Wassergehalt W von 50 Gew% bei 1700, während sie bei einem Wassergehalt von 70% bei 4500 liegt. Bei Wassergehalten zwischen 50 und 70% gelten linear interpolierte Werte, d. h. $P_w$ = W·150 - 5500. In einer besonders bevorzugten Ausführungsform liegt die untere Grenze von $P_w$ bei einem Wassergehalt W von 50 Gew% bei 1400, während sie bei einem Wassergehalt von 70% bei 4000 liegt, d. h. $P_w$ = W·130 - 5100. In einer ganz besonders bevorzugten Ausführungsform liegt die untere Grenze von $P_w$ bei einem Wassergehalt W von 50 Gew% bei 1200, während sie bei einem Wassergehalt von 70% bei 3500 liegt, d. h. $P_w$ = W·115 - 4550.

In einer bevorzugten Ausführung liegt die Viskosität des geschmolzenen Gels bei einer Temperatur im Bereich 40 bis 80°C, noch bevorzugter im Bereich 45 bis 70°C, am bevorzugtesten 50 bis 65°C im Bereich 60 - 2000 Pas, wobei im Sinne der Erfindung unter Viskosität die dynamische Viskosität zu verstehen ist.

Es handelt sich beim Gegenstand der Erfindung um ein nicht-chemisch, sondern physikalisch vernetzendes Gel auf Basis von PVA. Das efindungsgemässe Gel ist gut verarbeitbar, weist gegenüber herkömmlichen PVA Gelen ein beschleunigtes Aushärtungsverhalten auf und zeigt selbst unter massiven Belastungen, welche im biomechanischen Labor auf reparierte humane Bandscheiben appliziert wurden, keine Extrusion. Bei Steigerung der Belastung zerbrachen die Endplatten der Bandscheiben, ohne dass vorher Gel extrudiert hatte. Im Weiteren beschreibt die Erfindung Konzepte und Geräte, die geeignet und notwendig sind, um die PVA Gele in der Praxis einzusetzen.

Ebenso nimmt die Gelierungsgeschwindigkeit mit zunehmender Viskosität des Gels zu, was für einen erfolgreichen Einsatz essenziell ist, denn z.B. bei einem Einsatz im Bereich Nucleus Replacement sollte das Gel nach ca. 15min zumindest geringfügig belastbar sein.

PVA Mischungen mit Gewichtsmittel des Polymerisationsgrades $P_w$ innerhalb der definierten Grenzen können beispielsweise dadurch erhalten werden, dass ein Typ von PVA mit geeignetem $P_w$ eingesetzt wird oder zwei Typen mit verschiedenem $P_w$ und Anteilen, sodass die Kombination innerhalb der definierten Grenzen für $P_w$ liegt oder drei oder mehr Typen mit verschiedenem $P_w$ und Anteilen, sodass die Kombination innerhalb der definierten Grenzen für $P_w$ liegt. Bevorzugt weist die Mischungen mindestens zwei Typen mit unterschiedlichem $P_w$ auf, wobei der eine Typ, PVA1 ein Gewichtsmittel des Polymerisationsgrades $P_w$ von >1500, vorzugsweise >1700, noch bevorzugter >2000 und der andere Typ, PVA2 von <1500, vorzugsweise <1700, noch bevorzugter <2000 aufweist. Die untere Grenze für den Anteil von PVA1 in Gew.% bezogen auf PVA1 und PVA2 liegt bei 5%, vorzugsweise 20%, noch bevorzugter 30%, am bevorzugtesten > 35%. Die obere Grenze für den Anteil von PVA1 in Gew.% bezogen auf PVA1 und PVA2 liegt bei 60, vorzugsweise 55, noch bevorzugter 50, am bevorzugtesten 45.

Die mittlere Gelierungsgeschwindigkeit während der ersten 24 Stunden Aushärtungszeit kann mittels eines Kompressionstests bestimmt werden, wobei der E-Modul in Funktion der Zeit untersucht wird. Diese Gelierungsgeschwindigkeit in MPa pro Stunde ist bevorzugt > 0,01, vorzugsweise > 0,03, noch bevorzugter > 0,06, am bevorzugtesten > 0,08. Ganz besonders bevorzugt ist eine Gelierungsgeschwindigkeit > 0,10.

E-Modul in MPa der voll ausgehärteten PVA Gele liegt bevorzugt bei > 0,25, vorzugsweise >0,5, noch bevorzugter > 0,75, am bevorzugtesten > 1,0. Die obere Grenze für den E-Modul in MPa der voll ausgehärteten PVA Gele liegt bei < 10, vorzugsweise < 7,5, noch bevorzugter < 6,5, am bevorzugtesten < 5,5.

Eine wichtige Eigenschaft der PVA Gele für die Nucleus Anwendung ist das Rückstellverhalten nach längerer Belastung in Wasser oder Ringer Lösung. In einer bevorzugten Ausführungsform wird die Höhe eines PVA Zylinders, der mit 0,7MPa während 12h belastet wurde innerhalb der nächsten 4 Tage zu >70%, vorzugsweise zu >75%, noch bevorzugter zu > 80%, am bevorzugtesten > 83% wiederhergestellt.

Bevorzugt weisen die erfindungsgemäßen PVA Gele nach Gelierung bei 25°C einen Schmelzpeak gemessen im DSC (gemäss DIN 53765) mit einer Peak Temperatur (Temperatur des Maximums) im Bereich von 80 - 95°C auf. Weiterhin ist es bevorzugt, dass die PVA Gele nach Gelierung bei 25°C zusätzlich einen Schmelzpeak gemessen im DSC mit einer Peak Temperatur (Temperatur des Maximums) im Bereich von 45 - 65°C auf.

Charakterisierung des PVA

An die Polyvinylalkohole PVA1, PVA2 und gegebenenfalls weiteren Typen von PVA werden dieselben Anforderungen gestellt, welche eine möglichst gute Kristallisierbarkeit der PVA und eine hohe Stabilität der Kristallite ermöglichen. Somit sind Abweichungen von der idealen Struktur $[-CH_2-CHOH-]_n$ anteilmässig möglichst gering zu halten.

Der Hydrolysegrad (H) des PVA in mol% ist bevorzugt > 98, vorzugsweise > 99, noch bevorzugter > 99,2 noch bevorzugter > 99,4. In einer besonders vorteilhaften Ausführungsform ist H > 99,85, vorzugsweise > 99,9, noch bevorzugter > 99,95, am bevorzugtesten > 99,98. Im Bereich dieser hohen Hydrolysegrade hat eine nur geringe Steigerung einen erstaunlich deutlichen Effekt auf die Endeigenschaften des Gels.

Vorteilhaft ist ein Gehalt (G) an 1,2-Glycol in mol% von < 3, vorzugsweise < 1, noch bevorzugter < 0,5, am bevorzugtesten < 0,2.

Vorteilhaft ist eine Anzahl an Kurzkettenverzweigungen des PVA pro Monomereinheit von $< 10^{-2}$, vorzugsweise $< 10^{-3}$, noch bevorzugter $< 10^{-4}$, am bevorzugtesten $< 10^{-6}$.

Weitere Störungen der Regularität wie Carbonylgruppen in der Kette sind ebenfalls unerwünscht, bei üblichen PVA ist ihr Anteil mit typischerweise < 0,02mol% jedoch vernachlässigbar.

Bezüglich der Taktizität wird eine ataktische Konformation gegenüber einer isotaktischen bevorzugt, am bevorzugtesten ist eine syndiotaktische Konformation, bzw. ein möglichst hoher Anteil an syndiotaktischen Diaden. Die Taktizität von PVA wird durch die Art der Monomere festgelegt, womit das Vorläufer Polymer, aus dem das PVA erhalten wird, polymerisiert wird, sowie durch die Reaktionsbedingungen bei dieser Polymerisation, wobei mit abnehmender Temperatur während der Polymerisation der syndiotaktische Anteil zunimmt.

Wird das Vorläuferpolymer aus Vinylacetatderivaten der Art $CH_2=CHOCOR$ polymerisiert, wobei R beispielsweise H, $CH_3$, $C_3H_7$, $C_4H_9$, $CCIH_2$, $CCl_3$, $CF_3$ $C_4H_5F_4$, $C_6H_7F_6$, oder $C_6H_5$ sein kann, so nimmt der Anteil an syndiotaktischen Diaden mit dem Volumen der Gruppe R zu (während der 1,2-Glycolgehalt vorteilhaft abnimmt) und die im Vorläufer Polymer erhaltene Taktizität bleibt bei der nachfolgenden Hydrolyse zum PVA erhalten. Bevorzugt sind daher Monomere zur Polymerisation des Vorläufer Polymers wie Vinylacetat, Vinylchloroacetat, Vinyldichloroacetat, Vinylbromoacetat, insbesondere Vinyltrifluoroacetat.

Wird das Vorläuferpolymer aus aliphatischen Vinylsäureestern hergestellt, werden ebenfalls hohe Anteile an syndiotatkischen Diaden erhalten, während die resultierenden PVA ausserdem sehr niedrige 1,2-Glycol Gehalte aufweisen. Beispiele sind Vinylformat, Vinylpropionat, Vinylbutyrat, Vinylpivalat. Mittels Vinylpivalat können auch sehr hohe Molekulargewichte erreicht werden.

Vollhydrolysierte PVA erhalten aus Polyvinylacetat sind auch bei hohen Kristallisationsgraden bei 100°C in Wasser löslich, während vollhydrolysierte PVA deren Vorläufer Polymer aus Vinylacetatderivaten mit voluminöser Gruppe R (z.B. Vinyltrifluoroacetat) oder aus aliphatischen Vinylsäureestem (z.B. Vinylfromat, Vinylpivalat) hergestellt wurden infolge des geringen 1,2-Glycolgehalt und des hohen Anteils an syndiotaktischen Diaden selbst bei 100°C in unlöslicher Form erhalten werden können. Daraus wird die Bedeutung dieser Parameter für die Kristallisierbarkeit und die Stabilität der Kristallite deutlich.

Betreffend der Polydispersität P der drei zuvor erwähnten PVA Typen ist P < 5 bevorzugt, besonders bevorzugt < 3, ganz besonders bevorzugt < 2.

Bezüglich der Topologie sind übliche PVA vorwiegend linear, Langkettenverzweigungen treten bei üblichen PVA wenn überhaupt, dann selten auf. Nahezu vollständige oder vollständige Linearität wird bei kurzkettigen PVA2 bevorzugt, wobei diese Bedingung praktisch immer erfüllt ist, während langkettige PVA1 nicht notwendigerweise möglichst linear sein müssen, ein Anteil an Langkettenverzweigungen kann bei PVA1 sogar vorteilhaft sein, wenn die Länge dieser Seitenketten einen Polymerisationsgrad DP > 40 aufweist.

Weitere Polymere

Neben PVA kann das PVA Gel zur Modifikation der Eigenschaften und für spezifische Anwendungen weitere Polymere enthalten, bevorzugt synthetische Polymere, insbesondere Polycarbonate, Polyacrylate und Polymethacrylate, Polyethylenglycole, Polythylenoxide, Polyvinylpyrrolidone, Polycaprolactone oder Polymere natürlichen Ursprungs, bevorzugt Hydrokolloide und Polysaccharide, insbesondere Stärke und Stärke Derivate. Solche weitere Polymere werden bevorzugt zu Anteilen in Gew.% bezogen auf 100 Gew.% PVA Gel von 0 bis 30, besonders bevorzugt 0 bis 15, ganz besonders bevorzugt 1 bis 10 eingesetzt.

Zusatzstoffe

Als Zusatzstoffe werden einfache Füllstoffe und funktionale Füllstoffe bzw. Wirkstoffe oder Röntgenkontrast erzeugende Stoffe wie beispielsweise Bariumsulfat bezeichnet. Röntgenkontrast erzeugende Stoffe werden bevorzugt zu Anteilen in Gew.% von 10 bis 50, besonders bevorzugt von 25 bis 40, jeweils bezogen auf 100 Gew.% PVA Gel, eingesetzt.

Anwendungen

Da die erfindungsgemässen PVA Gele mit einem weiten Bereich von mechanischen Eigenschaften erhalten werden können, sind eine ganze Reihe von Anwendungen möglich. Einerseits können bisherige PVA Gele in allen Anwendungen vorteilhaft ersetzt werden, da die neuen Gele sehr viel einfacher herzustellen sind (Giessbarkeit, Gelbildungstemperatur > 0°C, Stabilität) und mindestens gleich gute mechanische Eigenschaften aufweisen. Andererseits eröffnen die neuen Gele, insbesondere als in situ gelierende Gele ausserordentlich interessante Anwendungen im Bereich der Biomedizin (z.B. Tissue und Scaffold Engineering) und insbesondere in der Orthopädie. Gewebe, Gele, Knorpel, insbesondere Sehnen, Bänder, Gelenk Oberflächen, Menisci, Nervenummantelungen, Hamröhren, Herzklappen, Gelkörper der Bandscheiben (Nucleus) können ersetzt oder repariert werden. Im Bereich der Bandscheiben und Rückenwirbel kommen insbesondere folgende spezifische Anwendungen in Frage: Total Disc Replacement, Nulceoplasty, Facet Replacement, Segment Replacement, Vertebroplasty, Kyphoplasty, Lordoplasty. Ganz besonders geeignet sind die erfindungsgemässen Gele für den Bereich Nucleus Replacement. Weitere Anwendungen liegen im Bereich der kosmetischen bzw. plastischen Chirurgie, wo die Gele eine Alternative zu Silikon Implantaten darstellen.

Applikationsverfahren

Zur Applikation des PVA Gels wird das Gel erfindungsgemäß nach einer Lagerungszeit nach einem Verfahren verarbeitet, das folgende Schritte aufweist:

a) Aufheizen während einer Zeit t1 auf eine Temperatur T1
b) Warten während einer Zeit t2 bei der Temperatur T1
c) Abkühlen während einer Zeit t3 auf eine Temperatur T2
d) Injektion bei einer Temperatur T3
e) Aushärtung bei Körpertemperatur

Das PVA Gel befindet sich während der Schritte a) und b) bevorzugt in einem geschlossenen Behälter, welcher während, oder nach Schritt c) geöffnet wird. Bei dem Behälter handelt es sich bevorzugt um eine Ampulle.

Aufheizen

Die Temperatur T1 ist genügend hoch, damit das PVA Gel mindestens teilweise, bevorzugt vollständig geschmolzen wird. In einer bevorzugten Ausführungsform liegt T1 in °C bei > 85, noch bevorzugter > 95, am bevorzugtesten > 97. Die Temperatur T1 kann erreicht werden, indem die Temperatur kontinuierlich auf T1 erhöht wird, oder durch aktive Regelung indem zunächst eine etwas höhere Temperatur erreicht und dann auf T1 abgekühlt wird.
Die Temperatur T1 kann auch auf einen Wert deutlich oberhalb 100°C eingestellt werden, da PVA eine gute Hitzebeständigkeit aufweist. In einer bevorzugten Ausführungsform ist T1 in °C < 200, vorzugsweise < 140, noch bevorzugter < 110, am bevorzugtesten < 105.
Die Temperatur T1 muss nicht notwendigerweise konstant sein, sie kann auch innerhalb der angegebenen Grenzen einen Verlauf aufweisen.
Die Zeit t1 kann an sich beliebig lange sein. In einer bevorzugten Ausführungsform ist t1 in min < 30, vorzugsweise < 20, noch bevorzugter < 15, am bevorzugtesten < 5.

Warten

Die Zeit t2 kann ebenfalls an sich beliebig lange sein, insbesondere kann das geschmolzene Gel auf T1 bereitgehalten werden. In einer bevorzugten Ausführungform ist t1 in min < 30, vorzugsweise < 20, noch bevorzugter < 10, am bevorzugtesten < 5. Die Zeit t2 kann insbesondere auch 0min betragen. Die Temperatur T1 kann während der Wartezeit t2 konstant sein oder einen Verlauf aufweisen.

Abkühlen

Die Temperatur T2 in °C liegt im Bereich 30 - 80, vorzugsweise 45 - 70, noch bevorzugter 50 - 65 am bevorzugtesten 50 - 60. Sie kann durch kontinuierliches Abkühlen oder durch aktive Regelung eingestellt werden.
In einer bevorzugten Ausführungsform ist die Abkühlzeit t3 in min < 30, vorzugsweise < 15, noch bevorzugter < 10, am bevorzugtesten < 5. Je kürzer die Abkühlzeit ist, umso länger kann die Injektionszeit sein, welche kritisch ist.

Injektion

Die Injektion erfolgt bei einer Temperatur T3, wobei T3 in °C im Bereich 30 - 80, vorzugsweise 45 - 70, noch bevorzugter 50 - 65, am bevorzugtesten 50 - 60 liegt.
Die gesamte Aufbereitungszeit des PVA Gels in min, beginnend mit dem Aufheizen und endend mit dem Ende der Abkühlung ist in einer bevorzugten Ausführungsform möglichst kurz und liegt bei < 30, vorzugsweise < 20, noch bevorzugter < 10, am bevorzugtesten < 5.

Behälter für das in situ gelierende PVA Gel

Die Erfindung betrifft weiterhin einen geschlossener Behälter enthaltend das erfindungsgemäße in situ gelierende PVA Gel. Bei dem geschlossenen Behälter handelt es sich bevorzugt um eine Ampulle.
Bevorzugt umfasst der geschlossene Behälter einseitige oder beidseitige Endkappen, eine Membrane, vorzugsweise eine Gummimembran, und einen Stempel, der gleitend abgedichtet ist. Die gleitende Abdichtung kann beispielsweise durch eine Ringdichtung oder einen O-Ring erfolgen. Eine Endkappe hindert den Stempel daran, aus der Ampulle herausgedrückt zu werden. Der Stempel weist eine zentrale Aussparung auf.

Injektionsvorrichtung

Außerdem betrifft die Erfindung eine Injektionsvorrichtung, umfassend eine an der Vorderseite der Injektionsvorrichtung montierte Kanüle, deren in den Innenraum der Injektionsvorrichtung vorstehende Ende schneidende Kanten aufweist, eine an der Hinterseite der Injektionsvorrichtung aufgesetzten Schraubkappe, welche ein zentrales Gewindeloch aufweist, einen Gewindestift der durch das zentrale Gewindeloch der Schraubkappe geführt ist, einen Handdrehknopf, der am Gewindestift angeordnet ist, wobei zwischen Vorderseite und Hinterseite der Injektionsvorrichtung ein Hohlraum ausgebildet ist, welcher einen erfindungsgemäßen geschlossenen Behälter aufnimmt.
Bevorzugt ist die Injektionsvorrichtung eingerichtet, um den Injektionsdruck beim Injizieren eines PVA Gels durch die Kanüle zu messen.

**Beispiele**

Analytische Methoden

Messung der mechanische Eigenschaften

Die mechanischen Eigenschaften wurden im Modus "unconfined compression" gemessen, d. h. die Ausdehnung des Probenkörpers war in der Richtung senkrecht zur Kompressionsrichtung nicht eingeschränkt.

Gerät: Instron 5542, Kraftmessdose: 500N. Software: Series IX, Automated Materials Testing System 50.00
Probenkörper: Zylinder mit Durchmesser von 11,8mm und einer Höhe von 12mm
Kompressionsgeschwindigkeit: 10mm/min
Temperatur: 25°C

Die Bestimmung des E-Moduls erfolgte durch mittlere Steigung der Spannungs-Kompressionskurve im Bereich 0,20 - 0,25mm/mm.

Rückstellverhalten (Recovery)

Zylindrische Gele mit einem Durchmesser von 11,8mm und einer Höhe H0 von 12mm wurden bei 25°C in Wasser (bzw. Ringer Lösung, wenn das Gel mit Ringer Lösung hergestellt wurde) während 4 Tagen gelagert, wonach die Höhe H1 gemessen wurde. Dann wurde eine Last von 0,7MPa appliziert (typische Last auf einer Bandscheibe) und 12h konstant gehalten (Belastung während dem Tag), wobei der Gelkörper komprimiert wurde. Danach wurde der Gelkörper entlastet und 4 Tage bei 25°C gelagert, wobei sich asymptotisch eine Höhe H2 einstellte. Die Rückstellung bzw. Recovery in % wurde erhalten als 100·H2/H0.

Wassergehalt

Der Wassergehalt von PVA Proben wurde durch Trocknen im Ofen bei 90°C über Phosphorpentoxid als Trocknungsmittel während 48h erhalten, wobei die Probengrösse jeweils < 3mm war. Der Wassergehalt wurde erhalten als Gewichtsverlust dividiert durch das ursprüngliche Gewicht.

DSC

Gerät: DSC 7 von Perkin Elmer,
Messung gemäss DIN 53765, 10°C/min.

Viskosität (dynamische Viskosität)

Für die Messung der Viskositäten wurden PVA Gele in einer Spritze zuerst 5min bei 100°C geschmolzen. Die Spritze hatte einen Querschnitt von 111mm$^2$ und einen Innendurchmesser von 11,9mm. Die Länge der Kanüle war 5 Inch (127mm) und hatte einen Innendurchmesser von Gauge 8 (3,2mm). Danach wurde die Spritze mit Inhalt in ein Wasserbad mit einer Temperatur von 50 bzw. 60°C gebracht und dort wiederum gelagert. Dann wurde der Inhalt mit 1,41mm/s während 8 Sekunden ausgestossen.

Druckmessung: MTS 858 Mini Bionix von MTS Systems Co. (Eden Prairie, Minnesota), weggesteuert, ausgestattet mit einer 10KN Kraftmessdose und gesteuert mit Testware-SX 4.0B.

Aus den gemessenen Drücken wurden mittels Kalibration dynamische Viskositäten erhalten. Die Kalibration wurde bei 25°C mit Viskositäts-Standards von Brookfield Engineering Laboratories Inc.(USA) und mit exakt dem gleichen Set-up wie für die PVA Gele durchgeführt. Die Viskositäts-Standards hatten bei 25°C Viskositäten in mPas von 95360, 201363, 381940, 546800.

Vergleichsbeispiele

Es wurden PVA Gele gemäß den Beispielen 2, 6, 7, 8 und 9 der WO 2006/021122 hergestellt. Hierzu wurde ein PVA1 (Polymerisationsgrad DPn = 2500, Pw1 = 5000) mit einem PVA 2 (DPn = 180; Pw2 = 360 bzw. DPn = 300, Pw2 = 600) versetzt. Der Anteil an PVA1 bezogen auf PVA1 plus PVA2, das Gewichtsmittel des Polymerisationsgrades Pw des PVA, der Wassergehalt der erhaltenen Gele, und die Viskosität der Gele bei 50°C sind Tabelle 2 zu entnehmen. Es ist zu erkennen, dass die Vergleichsgele zwar einen Wassergehalt gemäß der Erfindung aufweisen, sich jedoch bezüglich des Pw von erfindungsgemäßen Gelen unterscheiden. Dies führt zu sehr niedrigen Viskositäten, die in Tabelle 2 ebenfalls aufgeführt sind, so dass die Vergleichsgele beim Einsatz als Nucleus Ersatz aus der Kavität hinausfliessen würden, bevor sie genügend geliert sind, um das Fliessen zu verhindern.

Beispiel 1

30,05g PVA1 (Pw = 4300) mit einem Wassergehalt von 8,8 Gew.% wurde mit 106,46g Ringer Lösung bei 98°C in einem abgeschlossenen Kolben unter Rühren während 50min gelöst. Dann wurde bei gleich bleibender Temperatur 38,850g PVA2 (Pw = 600) mit einem Wassergehalt von 6,6% zugegeben und die Mischung während 25min gerührt, bis die Mischung homogen und vollständig gelöst war. Der Anteil von PVA1 bezogen auf PVA1 plus PVA2 lag bei 40,3 Gew.%, der Wassergehalt nominal bei 62,2 Gew.% und real (infolge von Verlust) bei 61 Gew.%. Dann wurde 34g Bariumsulfat Pulver zugemischt, resultierend zu 16,8 Gew.% Bariumsulfat bezogen auf die gesamte Mischung und die Mischung wurde durch Rühren homogenisiert. Anschliessend wurde die Mischung in Reagenzgläser abgefüllt und die Reagenzgläser wurden zentrifugiert und luftdicht verschlossen. In diesem Zustand wurde das Gel gelagert. Für die weitere Verarbeitung wurde das Gel im Reagenzglas bei rund 98°C geschmolzen und entnommen, bzw. zur Herstellung von Zylindern für die Analyse der mechanischen Eigenschaften oder für die Injektion in ausgeräumte Bandscheiben für Kadaver Versuche.

Die Viskosität des Gels nach Schmelzen bei 100°C und nach 5min Lagerung bei 60°C lag bei 280 Pas. Die Aushärtungsgeschwindigkeit während den ersten 24h gemessen am E-Modul in unconfined Kompression und bei 37°C lag bei 0,027MPa/h und der E-Modul in unconfined Kompression nach vollständiger Aushärtung bei 37°C lag bei rund 3MPa. Die Rückstellung (Recovery) lag bei 77%.

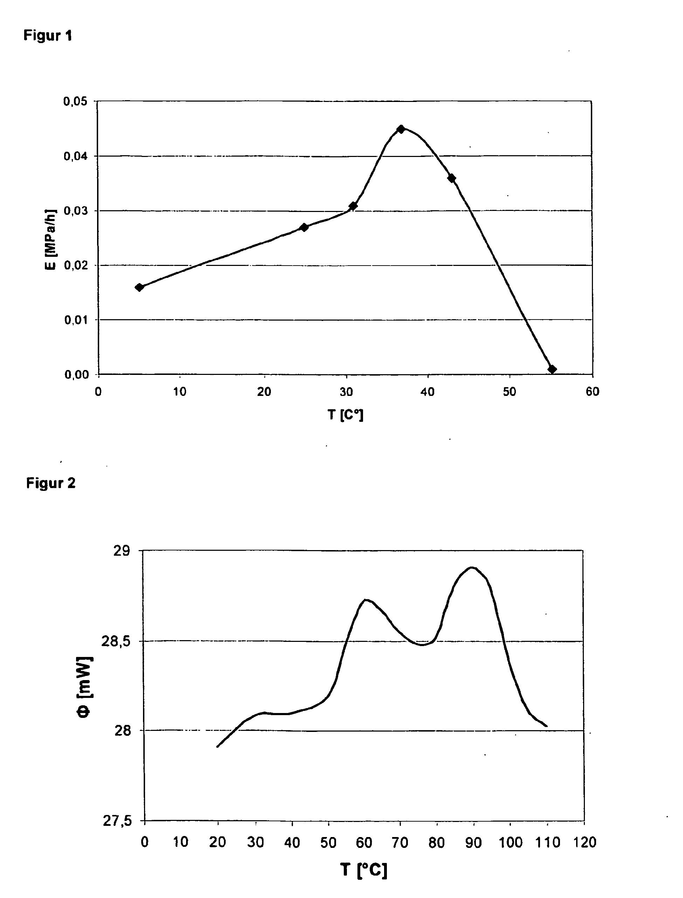

Das Ergebnis einer Messung des E-Moduls eines Gels, das analog hergestellt worden ist, jedoch mit einem Wassergehalt von 60.1%, ist in Figur 1 dargestellt. Es ist ersichtlich, dass die Aushärtung bei 37°C im Bereich der maximalen Aushärtungsgeschwindigkeit lag.

Für die Kadaver Versuche wurden Bandscheiben ausgeräumt, mittels 6mm Dilatationstechnik die Kanüle zur Injektion des PVA eingeführt und so dass PVA Gel injiziert. Die Bandscheiben wurden in physiologischer Kochsalzlösung 24h gelagert, bevor die mechanische Belastung appliziert wurde. Das biologische System wurde durch Antibiotika stabilisiert. Die mechanische Belastung lag bei 1400N axiale Last (entspricht rund 1,5MPa Druck auf die Bandscheibe) und einer Biegung bis 2/3 von physiologischer Biegung. Modus war alternierend zwischen Flexion-Kompression, Seitliche Biegung-Kompression, Extension-Kompression. Bei keiner der untersuchten Bandscheiben wurde Extrusion beobachtet. Die Resultate sind in Tabelle 1 aufgeführt.

Beispiel 2

Mit analoger Methode wie in Beispiel 1 wurde das Gel PVA 92 hergestellt, wobei jedoch der Anteil PVA2 bezogen auf PVA1 und PVA2 bei 31,4%, der Wassergehalt nominal bei 62,9 und real, infolge von Verdampfungsverlusten bei der Herstellung, bei 59,1 lag und kein Füllstoff eingesetzt wurde. Die Viskosität des Gels nach Schmelzen bei 100°C und nach 5min Lagerung bei 60°C lag bei 190 Pas. Das Gel wurde anschliessend 4 Wochen bei Raumtemperatur gelagert und dann mittels DSC untersucht. Die DSC Kurve ist in Figur 2 dargestellt. Das Probengewicht lag bei 18,37mg, die Heizgeschwindigkeit bei 10°C/min und es wurden zwei Peaks mit Peak Temperaturen bei 59,9 und 92,1 festgestellt.

Beispiel 3

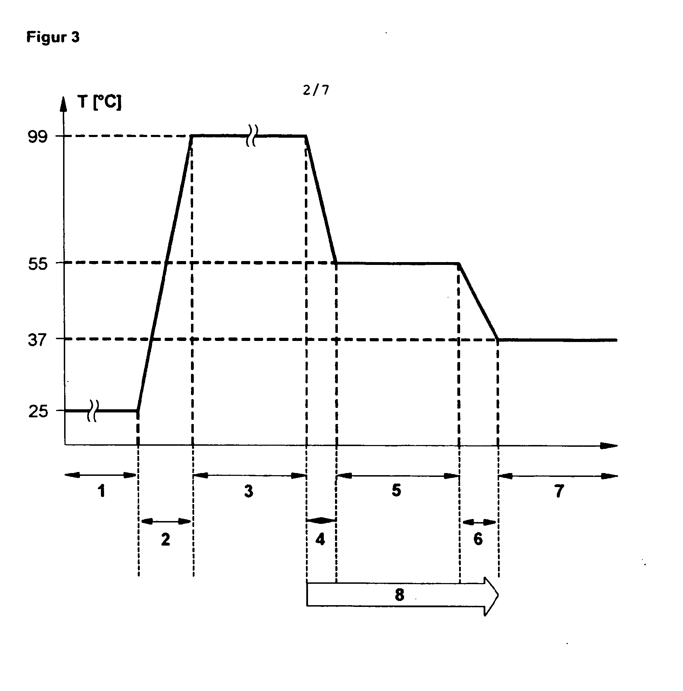

Figur 3 zeigt, wie ein erfindungsgemäßes PVA Gel im festen Zustand transportiert und gelagert (typisch für Wochen bis Monate, 1) wird. Kurz vor der Operation wird das Gel für kurze Zeit (typisch für 5min - 15min) bei Temperaturen typisch auf 95°C- 99°C, 2) verflüssigt. Bei Temperaturen unter dem Siedepunkt von Wasser entstehen im zu schmelzenden Gel nie relevante Überdrücke, was geringere Anforderungen an die Dichtigkeit des jeweiligen Gefässes stellt. Bei erhöhter Temperatur (über etwa 80°C, typisch bei 95°C - 99°C), kann das geschmolzene, aber abgedichtete Gel über längere Zeit (bis mehrere Stunden) zur weiteren Verarbeitung bereit gehalten werden (3).

Sobald, gemäss Verlauf der Chirurgie, die Injektion des Gels kurz bevor steht (typisch nicht mehr als 5 Minuten), wird eine möglichst rasche Abkühlung des Gels auf eine für den Körper verträgliche Temperatur eingeleitet (typisch auf 50°C - 60°C, 4). Nach der Übertragung in eine Spritze kann die Injektion in den Körper (z.B. in die Bandscheibe, den Wirbelkörper, etc., 5) beginnen. Nach Injektion in den Körper kühlt sich das Gel bis auf 37°C Körpertemperatur ab (6) und wird hierbei zunehmend fester. Nach Erreichen der gewünschten Festigkeit nimmt das Gel seine dauerhafte Funktion als mechanisches Implantat wahr (7).

Das Verarbeitungsintervall (8) ist zeitkritisch, muss also innerhalb einer vorgegeben Zeitspanne durchgeführt werden (typisch innerhalb von 5min -15min).

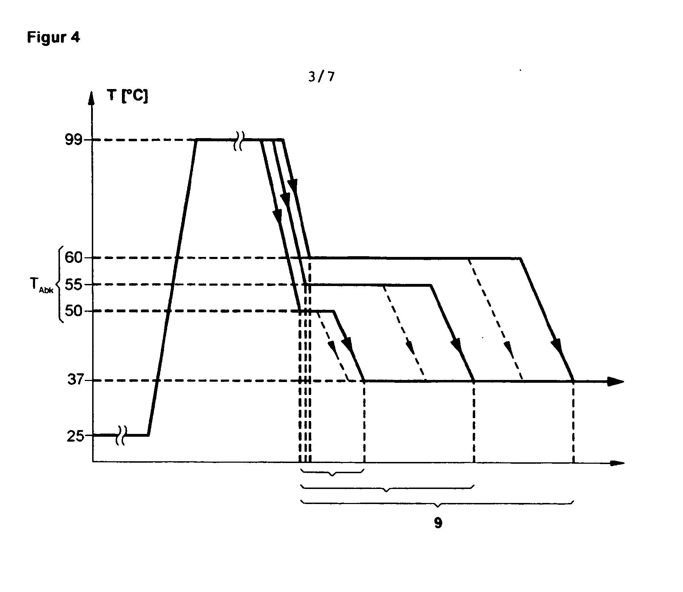

Für das geschmolzene PVA Gel beginnt mit dem Einleiten der Abkühlung (4) auf eine für den Körper verträgliche Temperatur $T_{Abk}$ eine zeitkritische Phase. Figur 4 zeigt, dass die maximal mögliche Zeit (9), die für die Injektion (5) zur Verfügung steht, stark von $T_{Abk}$ abhängt. Veränderungen von wenigen °C (typisch +/- 2°C - 5°C) können die Verarbeitungszeit um mehrere Minuten verlängern oder verkürzen. Diese Abhängigkeit kann zur Optimierung der Verarbeitungszeit (5) nach praktischen Gesichtspunkten der Chirurgie verwendet werden. Diese sollte im Normalfall etwa 5min - 15min betragen, könnte aber bei Bedarf in einem viel weiteren Bereich (z.B. 10sec - 60min) variiert werden.

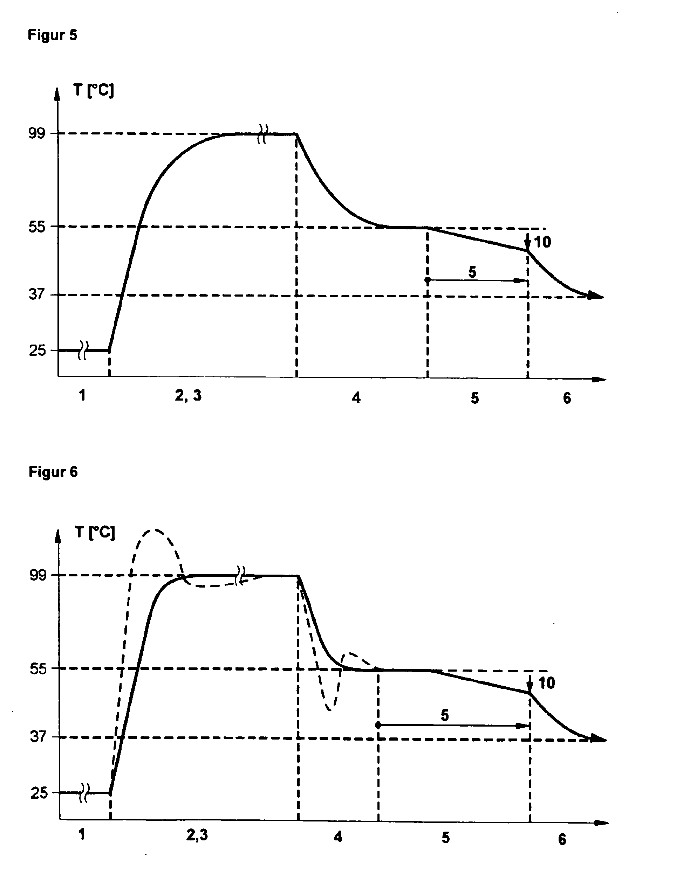

Die verschiedenen applizierten Temperaturschritte, die zum Vorbereiten und Einspritzen des PVA Gels notwendig sind, werden mit einer dafür spezifisch vorgesehenen Apparatur erreicht. Figur 5 beschreibt die Wirkungsweise einer Apparatur, die Heizelemente (oder Kühlelemente) mit einer fixen Temperatur einsetzt (z.B. stabilisierte elektrische Heizquelle, Kühlaggregat, Peltier Element, Wasserbad). Die jeweilige Zieltemperatur des Gels wird langsam und asymptotisch erreicht.

Figur 6 hingegen beschreibt die Wirkungsweise eine Apparatur mit intelligenter Temperaturregelung. Dank aktiver Regelung mit überschiessender Temperatur des Heizelementes (oder Kühlelementes) wird die jeweilige Zieltemperatur rascher erreicht. Dies kann zum Beispiel erreicht werden durch einen elektronischen Regelkreis mit Rückmeldung des Temperatur Ist-Wertes und einer entsprechenden aktiven Nachregelung um den Soll-Wert möglichst rasch zu erreichen. Ebenso resultiert eine gute Isolation des erhitzten Gels in der Injektionsspritze gegenüber der Umgebungstemperatur in einem kleineren Temperaturabfall (10) des Gels während der Einspritzzeit (5). Der kombinierte Vorteil der rascheren Erreichens der Einspritztemperatur dank intelligenter Temperaturregelung, sowie der guten Isolation des erhitzten Gels in der Spritze ist eine längere maximale Einspritzzeit (5) mit einer möglichst gleichbleibenden Viskosität des einzuspritzenden Gels.

Beispiel 4

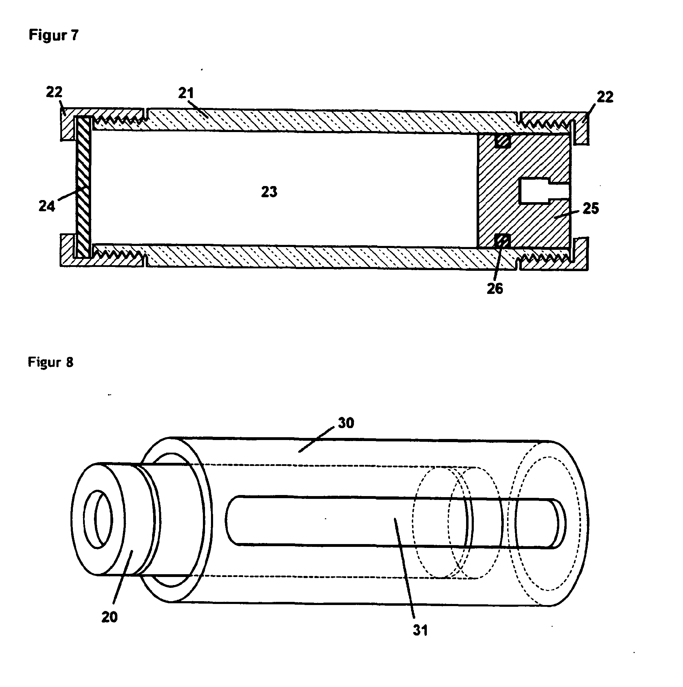

Das PVA Gel wird zur längeren Lagerung und für den Transport vor der Verwendung aufbewahrt in einer möglichst wasser- und gasdicht verschlossenen (sterilen) Ampulle (20). Diese ist in Figur 7 gezeigt. Die Einfüllung soll dabei möglichst komplett, aber insbesondere frei von Luftblasen sein. Die Ampulle (20) ist vorzugsweise für den Einweggebrauch bestimmt und hat eine zylindrische Form. Im Idealfall ist die Ampulle (20) aus einem transparenten Material (21) gefertigt, damit dessen Inhalt visuell kontrolliert werden kann. Ein- oder beidseitige Endkappen (22) weisen einen Schraub- oder Schnappverschluss auf.

Die Ampulle ist so konstruiert, dass sie sowohl für die Lagerung und den Transport des wasser- und gasdicht verschlossenen Gels (23), für das initiale Schmelzen des Gels, sowie zur späteren Einspritzung des Gels in den Körper unter hohem Druck (bis zu 20 MPa) verwendet werden kann. Die Ampulle (20) ist zu diesem Vielfachzweck konzipiert als eine Art hermetisch abgeschlossene, (steril) vorgefüllte Spritze.

Eine Seite der Ampulle (20) hat eine Gummimembrane (24) (ähnlich jener, die eine Ampulle üblicherweise für das wiederholte Aufziehen von Medikamenten in eine Spritze benötigt), die zum Ausdrücken des Gels aus der Ampulle mit der Kanüle zentral durchstochen wird. Die andere Seite der Ampulle ist verschlossen mit einem gleitend abgedichteten Stempel (25) (Ringdichtung (26) oder O-Ring). Die Endkappe (22) hindert den Stempel (25) daran, aus der Ampulle herausgedrückt zu werden (positiver Stop). Nach Einsetzen der Ampulle in die Spritze (40) kann der Stempel (25) durch das zentrale Loch des Endkappe (22) mit einem Gewindeschaft (41) nach vorne gestossen werden, wodurch das Gel aus der Ampulle auspresst wird. Der Gewindeschaft rastet hierbei im Loch des Stempels ein, damit beim entgegengesetzten Drehen des Gewindeschaftes der Stempel auch wieder zurückgezogen werden kann.

Figur 8 zeigt ein in etwa gleich langes Rohr (30), dessen Innendurchmesser (ID) nur knapp grösser ist als der Aussendurchmesser (OD) der Ampulle (20) ist, welches aus einem gut wärmespeichernden Material gefertigt (z.B. Messing, Kupfer). Die Wandung des Rohres (30) ist etwa 2mm - 5mm dick. Die erhitzte Ampulle wird zur besseren Wärmespeicherung des Gels im Innem des ähnlich temperierten Rohres aufbewahrt. Ein oder mehrere Längsschlitz(e) (31) am Rohr erlauben einen Einblick auf den Füllstand der Ampulle (20). Das Rohr dient zusätzlich als mechanische Verstärkung der (dünnwandigen) Ampulle, damit diese ohne starke Deformation (z.B. radiale Ausbuchtung) grösseren inneren Drücken (bis 20MPa) widerstehen kann.

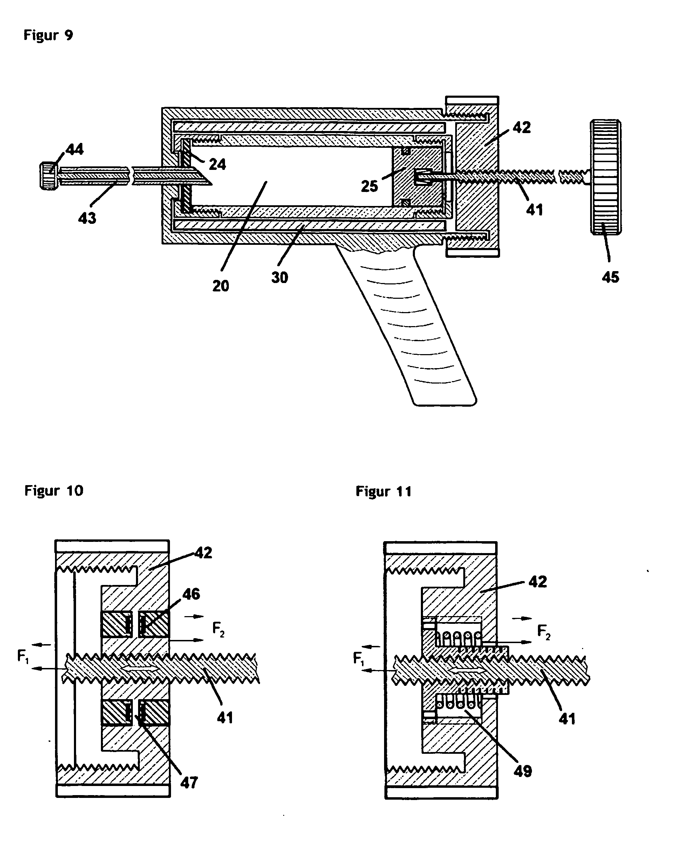

Figur 9 zeigt, wie die erhitzte und alsdann auf Einspritztemperatur abgekühlte Ampulle (20) im ebenfalls auf Einspritztemperatur vorgewärmten Rohr (30) (oder vorgewärmt auf wenige °C darüber) eingesetzt wird. Beide werden von hinten in die aufgeschraubte Spritze (40) (Schraubkappe (42) wurde entfernt) eingesteckt.

Eine konzentrisch am vorderen Ende der Spritze (40) montierte Kanüle (43) steht um etwa 5mm - 10mm in den Innenraum der Spritze vor. Ein innerer Einsatz (44) ("Trocar"), der von vorne in die Kanüle eingesteckt wird, schient die Kanüle innerlich zusätzlich und verhindert zudem deren Verstopfen. Das im Innenraum der Spritze vorstehende Teil der Kanüle ist schräg angeschliffen und hat schneidende Kanten. Beim Aufsetzen und Anziehen der von hinten aufgesetzten Schraubkappe (42) wird die Gummimembrane (24) der hermetisch abgeschlossenen Ampulle (20) mit der angeschliffenen Kanüle (43) durchstochen.

Der Gewindeschaft (41), der vom zentralen Gewindeloch der Schraubkappe geführt ist, wird mit dem gerändelten Handdrehknopf (45) nach vorne geschoben, bis dass er in der entsprechenden zentralen Aussparung des beweglichen Stempels (25) einrastet. Nachdem der innere Einsatz (44) aus der Kanüle entfernt wurde, kann mit dem Handdrehknopf (45) die Ampulle (20) mit-dem beweglichen Stempel (25) durch die (lange) Kanüle (43) ausgepresst werden. Die benötigte Kanülenlänge und -dicke ist abhängig von der Lokalität der Einspritzung. Für eine typische Anwendung zur Einspritzung von Gel in die humane Bandscheibe oder den humanen Wirbelkörper ist die Kanüle (43) zwischen 10cm und 15cm lang, und ist zwischen etwa 2mm und 5mm dick. Die benötigten Drücke um das relativ hochvisköse Gel durch eine lange und dünne Kanüle auszudrücken sind hoch, typischerweise von etwa 1MPa bis 20MPa.

Eine Druckmessung während der Einspritzung des Gels ist aus verschiedenen Gründen wünschbar. Die korrekte Arbeitsweise der Spritze (40) kann kontinuierlich überwacht werden. Zu grosse Drücke, welche die strukturelle Festigkeit der Spritze gefährden, können verhindert werden. Die gleichbleibende Füllung einer Kavität im Körper (z.B. eine ausgeräumte Bandscheibe) kann mit dem Fülldruck standardisiert werden. Die Figuren 10 und 11 zeigen, wie eine solche Druckmessung zum Beispiel im Innem der Schraubkappe eingebaut werden kann.

Die axiale Kraft, die via Gewindestift (41) der Spritze (40) auf den gleitenden Stempel (25) der Ampulle (20) übertragen wird, ist proportional zum erzeugten Druck in der Ampulle. Die Schraubkappe (42) bietet sich daher an, die axiale Kraft $F_1$ auf dem Gewindestift (41) einfach und direkt (als reaktive Kraft $F_2$) zu messen. Figur 10 beschreibt eine Kraftmessung mittels Dehnungsmessstreifen (DMS) (46), die in der Schraubkappe (42) auf einer dünnen Brücke (47) aufgebracht sind. Jeweils ein DMS Paar liegen sich gegenüber, so dass einer Dehnung und der andere Kompression misst. Zwei DMS Paare sind zu einer Temperatur kompensierten Vollbrücke zusammengeschaltet (Wheatstone Brücke). Das resultierende elektrische Signal (mV) ist proportional zur Kraft, muss aber üblicherweise elektrisch verstärkt und individuell geeicht werden für die jeweilige Messbrücke. Die Messung ist aber präzise, hat eine gute Auflösung und die Messvorrichtung ist in axialer Richtung sehr steif.

Figur 11 ist rein mechanisch, braucht also weder eine Spannungsquelle noch einen elektronischen Schaltkreis. Eine Druckfeder (48) wird bei axialer Last auf die Schraubkappe (42) komprimiert. Ein innerer Teil der Schraubkappe (42), der mit einer Nut gegen das Verdrehen gesichert ist, dient als Widerlager für den Gewindestift (41). Wenn die Feder (48) durch den Gewindestift (41) verkürzt wird, schiebt sich der innere Teil der Schraubkappe relativ zur äusseren Schraubkappe zunehmend vor. Die Kompressionskraft kann mittels einer geeichten Graduierung am inneren Teil direkt abgelesen werden. Die rein mechanische Messvorrichtung ist in axialer Richtung weit weniger steif jene in Figur 10, was die kontrollierte und fein dosierte Ausstossung des hochviskösen Gels aus der Ampulle erheblich erschwert.

Eine Vorrichtung mit zwei Heizelementen (A und B) dient zum kontrollierten Erwärmen und Abkühlen der mit PVA Gel gefüllten Ampulle und des dazu passenden wärmespeichernden Rohres. Die Heiz- und Kühlelemente (z.B. Peltier Elemente, elektrische Heizelemente und Kühlaggregate) können individuell angesteuert werden. Ein Temperatursensor, angebracht zum Beispiel auf dem Boden einer Aussparung und daher in direktem Kontakt jeweils mit der eingesetzten Ampulle oder dem Rohr, dient zur Rückmeldung der Ist-Temperatur. Eine Temperatursteuerung vergleicht die Ist-Temperatur mit der Soll-Temperatur.

Die einfache Bedieneroberfläche startet mit einer Taste das initiale Erhitzen und Schmelzen des Gels in der Ampulle. Eine Anzeige (optisch und/oder akustisch) signalisiert das Erreichen der Schmelztemperatur und damit die Gebrauchsbereitschaft des Gels für die baldige Einspritzung. Mit einer weiteren Taste kann das Abkühlen des Gels vor der Einspritzung eingeleitet werden. Von nun an ist das weitere Prozedere zeitkritisch.

Nach Erreichen der Einspritztemperatur zeigt eine Anzeige den möglichen Start des Einspritzvorganges an. Die Ampulle wird nun der Heizvorrichtung entnommen und zusammen mit dem wärmespeichernden Rohr in die Spritze eingesetzt. Das Zusammensetzen der Spritze erfolgt in jedem Fall im sterilen Bereich des Operationsfeldes. Gleichzeitig startet eine Stoppuhr rückwärts zu laufen. Sobald die Zeit Null erreicht, indiziert eine Wamanzeige, dass die Einspritzung nun beendet werden muss.

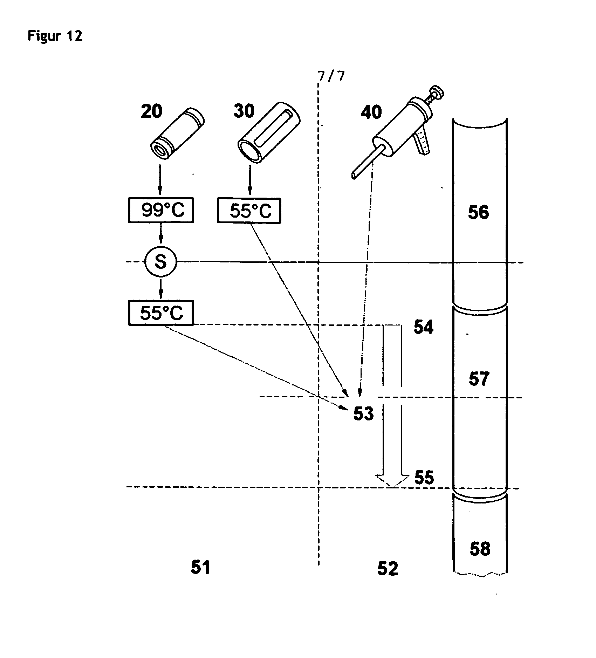

Figur 12 ist eine Zusammenfassung der Verwendung des Gels für die Einspritzung in eine Körperhöhle (hier gezeigt für die Einspritzung in eine humane Bandscheibe). Initial werden im nicht sterilen Bereich (51) des Operationsfeldes das Gel in der Ampulle (20) geschmolzen und das wärmespeichernde Rohr (30) auf Einspritztemperatur (oder leicht darüber) gebracht. Zwischenzeitlich wir die chirurgische Injektion des Gels vorbereitet.

Sobald die Einspritzung des Gels verlässlich auf weniger als 5min entfernt vorausgesagt werden kann, wird die kontrollierte Abkühlung des Gels auf Einspritztemperatur eingeleitet (Start S). Beim Erreichen der Einspritztemperatur wird die Spritze (40) im sterilen Bereich (52) des Operationsfeldes mit der Gel-gefüllten Ampulle und dem vorgewärmten Rohr geladen (53). Das Transfer der Ampulle und des Rohres aus dem unsterilen in den sterilen Bereich kann technisch einfach gelöst werden, zum Bespiel mit dem Öffnen und Auskippen der Ampulle und des Rohres aus einem inwendig sterilen Container.

Mit entsprechender Bedienerführung (Stoppuhr, Wamanzeige läuft zwischen Start Einspritzen (54) und Stopp Einspritzen (55)) kann das Gel nun während einer optimal eingestellten Verarbeitungszeit kontrolliert appliziert werden. Nach Ablauf der Verarbeitungszeit soll typischerweise die Kanüle noch kurze Zeit in der Wunde belassen werden (zum Beispiel für 1min bis 5 min), damit beim Herausziehen der Kanüle das weiter aushärtende Gel nicht rückwärts fliessen kann.

Es finden hierbei folgende Schritte statt: Zugang zur Bandscheibe, Ausräumen des Nucleus und Vorbereitung für die Injektion (56), Einspritzen und Röntgen zur Kontrolle (57) und Aushärten lassen und Kanüle zurückziehen (58).

**Bezugszeichenliste**

1:     Verfahrensschritt "Lagerung"

2:     Verfahrensschritt "Schmelzen"

3:     Verfahrensschritt "Warten"

4:     Verfahrensschritt "Abkühlen vor der Injektion"

5:     Verfahrensschritt "Injektion"

6:     Verfahrensschritt "Abkühlen nach der Injektion"

7:     Verfahrensschritt "Implantatnutzung"

8: Verarbeitungsintervall

9: maximal mögliche Zeit, die für die Injektion zur Verfügung steht

10: Temperaturabfall des Gels

20: Ampulle

21: transparentes Material

22: Endkappe

23: PVA Gel

24: Gummimembrane

25: gleitend abgedichteter Stempel

26: Ringdichtung

30: Rohr

31: Längsschlitz

40: Spritze

41: Gewindeschaft

42: Schraubkappe

43: Kanüle

44: innerer Einsatz

45: Handdrehknopf

46: Dehnungsmessstreifen

47: Brücke

48: Feder

51: nicht steriler Bereich

52: steriler Bereich

53: Zusammensetzen im sterilen Bereich

54: Start Einspritzen

55: Stopp Einspritzen

56: Zugang zur Bandscheibe, Ausräumen des Nucleus und Vorbereitung für die Injektion

57: Einspritzen und Röntgen zur Kontrolle

58: Aushärten lassen und Kanüle zurückziehen

**Tabelle 1**

| Nr. | Bandscheibensegment | Nucleus Material entnommen [g] | PVA Gel injiziert [cm$^3$] | Zeitdauer der Belastung [h] | Anzahl Belastungs Zyklen | Extrusion [%] |
|---|---|---|---|---|---|---|
| 1 | T11/12 | 2.36 | 2.64 | 48 | 69'300 | 0 |
| 2 | L3/4 | 4.31 | 4.20 | 15 | 20'883 | 0* |
| 3 | T12/L1 | 2.52 | 3.76 | < 5 | 5'446 | 0* |
| 4 | L2/3 | 4.37 | 3.78 | 44 | 60'874 | 0 |
| 5 | L2/3 | 4.14 | 3.95 | 48 | 70'000 | 0 |
| *Vorzeitiger Abbruch des Versuchs infolge Endplatten Fraktur. | | | | | | |

**Tabelle 2**

| Nr. | Pw1 | Pw2 | Pw | PVA1/PVA [%] | W [%] | Viskosität [Pas] |
|---|---|---|---|---|---|---|
| | | | | | | |
| 2 | 2500 | 180 | 749,76 | 8,4 | 54 | 25 |
| 6 | 2500 | 180 | 870,4 | 11 | 55 | 34 |
| 7 | 2500 | 300 | 1084 | 11 | 55 | 49 |
| 8 | 2500 | 180 | 1148,8 | 17 | 60 | 27 |
| 9 | 2500 | 300 | 1348 | 17 | 60 | 45 |

**Patentansprüche**

1. In situ gelierendes Polyvinylalkohol (PVA) Gel, **dadurch gekennzeichnet, dass**

   a) das PVA Gel PVA (Komponente i) und Wasser (Komponente ii) als Quellungsmittel aufweist,
   b) die Viskosität (dynamische Viskosität) des geschmolzenen Gels bei einer Temperatur im Bereich von 30 bis 100°C im Bereich von 60 bis 2000 Pas liegt,
   c) der Wassergehalt W des PVA Gels im Bereich von 52 bis 64 Gew.% liegt,
   d) die obere Grenze für das Gewichtsmittel des Polymerisationsgrades Pw des PVA durch die Beziehung Pw = W·150 - 5500 gegeben ist, und
   e) die untere Grenze für das Gewichtsmittel des Polymerisationsgrades Pw des PVA durch die Beziehung Pw = W·90 - 3800 gegeben ist.

2. In situ gelierendes PVA Gel nach einem der vorangehenden Ansprüche, **dadurch gekennzeichnet, dass** das PVA Gel mindestens zwei PVA-Typen PVA1 und PVA2 von PVA aufweist, wobei PVA1 ein Gewichtsmittel des Polymerisationsgrades Pw von > 1500 und PVA1 von < 1500 aufweist und der Anteil von PVA1 in Gew.%, bezogen auf die gesamte Menge an PVA1 und PVA2, im Bereich 5 bis 60 Gew.-% liegt.

3. In situ gelierendes PVA Gel nach einem der vorangehenden Ansprüche 1 oder 2, **dadurch gekennzeichnet, dass** das PVA Gel mindestens einen Füllstoff aufweist, der Röntgenkontrast erzeugt.

4. Geschlossener Behälter, enthaltend das in situ gelierende PVA Gel nach einem der vorangehenden Ansprüche 1 bis 3, wobei es sich bei dem geschlossenen Behälter bevorzugt um eine Ampulle (20) mit sterilem Innenraum, handelt.

5. Geschlossener Behälter nach Anspruch 4, umfassend einseitige oder beidseitige Endkappen (22), eine Membrane (24), und einen Stempel (25), der gleitend abgedichtet ist (26), wobei der Stempel (25) eine zentrale Aussparung aufweist.

6. Injektionsvorrichtung (**40**), umfassend

- eine an der Vorderseite der Injektionsvorrichtung (**40**) montierte Kanüle (**43**), deren in den Innenraum der Injektionsvorrichtung (**40**) vorstehendes Ende schneidende Kanten aufweist,
- eine an der Hinterseite der Injektionsvorrichtung aufgesetzten Schraubkappe (**42**), welche ein zentrales Gewindeloch aufweist,
- einen Gewindestift (**41**) der durch das zentrale Gewindeloch der Schraubkappe (**42**) geführt ist,
- einen Handdrehknopf (**45**), der am Gewindestift (**41**) angeordnet ist,

wobei zwischen Vorderseite und Hinterseite der Injektionsvorrichtung (**40**) ein Hohlraum ausgebildet ist, welcher einen geschlossenen Behälter nach einem der Ansprüche 4 oder 5 aufnimmt.

7. Injektionsvorrichtung (**40**) nach Anspruch 6, **dadurch gekennzeichnet, dass** sie eingerichtet ist, um den Injektionsdruck beim Injizieren eines PVA Gels durch die Kanüle (**43**) zu messen.

8. Verfahren zur Applikation eines in situ gelierenden PVA Gels, **dadurch gekennzeichnet, dass** das Verfahren folgende Schritte aufweist:

a) Schmelzen des Gels (**1**)
b) gegebenenfalls Warten (**2**)
c) Abkühlung des Gels (**3**)
d) Injektion des Gels (**4**)
e) Gelierung (**5**)

9. Verfahren nach Anspruch 8, **dadurch gekennzeichnet, dass** das PVA Gel ein PVA Gel gemäß einem der Ansprüche 1 bis 3 ist.

10. Verfahren nach Anspruch 9, **dadurch gekennzeichnet, dass** sich das PVA Gel in den Schritten a) und b) in einem geschlossenen Behälter nach einem der Ansprüche 4 oder 5 befindet, der während oder nach Schritt c) geöffnet wird.

11. Verfahren nach Anspruch 10, **dadurch gekennzeichnet, dass** es mittels einer Injektionsvorrichtung nach einem der Ansprüche 6 oder 7 durchgeführt wird.

12. Verfahren nach einem der Ansprüche 8 bis 11, **dadurch gekennzeichnet, dass**

- das PVA Gel in Schritt a) bei einer (Temperatur > 85°C geschmolzen wird,
- das PVA Gel in Schritt c) auf eine Temperatur im Bereich von 30 bis 80°C abgekühlt wird und die Abkühlzeit insbesondere < 30min beträgt, und
- das PVA Gel in Schritt d) bei einer Temperatur im Bereich von 30 bis 80°C injiziert wird.

13. Verfahren nach einem der Ansprüche 8 bis 12, **dadurch gekennzeichnet, dass** während der Injektion in Schritt d) ein Druck von < 20MPa erreicht wird.

14. Verfahren nach einem der Ansprüche 8 bis 13, **dadurch gekennzeichnet, dass** die Injektion in Schritt d) mittels einer Kanüle erfolgt und die Kanüle zur Injektion des PVA Gels mittels eines Dilatationsverfahrens in den Annulus eingeführt wird.

15. Verwendung des PVA Gels nach einem der Ansprüche 1 bis 3 in der Biomedizin, insbesondere in der Orthopädie, vorzugsweise bei Bandscheiben betreffend Nucleus Ersatz.

**Claims**

1. A polyvinyl alcohol (PVA) gel, gelling in situ, **characterized in that**

a) the PVA gel comprises PVA (component i) and water (component ii) as swelling agent,

b) the viscosity (dynamic viscosity) of the melted gel at a temperature in a range of 30 to 100 °C is in a range of 60 to 2000 Pas,

c) the water content W of the PVA gel is in a range of 52 to 64 % by weight,

d) the upper limit for the weight average of the degree of polymerisation Pw of the PVA is given by the relationship of Pw = W·50 - 5500, and

e) the lower limit for the weight average of the degree of polymerisation Pw of the PVA is given by the relationship of Pw = W·90 - 3800.

2. The PVA gel gelling in situ according to any one of the preceding claims, **characterized in that** the PVA gel comprises at least two PVA types, PVA1 and PVA2 of PVA, wherein PVA1 has a weight average of the degree of polymerisation Pw of > 1500 and PVA2 of < 1500, and the proportion of PVA1 in % by weight, in relation to the entire amount of PVA1 and PVA2, is in a range of 5 to 60 % by weight.

3. The PVA gel gelling in situ according to any one of the preceding claims 1 or 2, **characterized in that** the PVA gel comprises at least one filler which produces X-ray contrast.

4. A closed vessel, containing the PVA gel gelling in situ according to any one of the preceding claims 1 to 3, wherein the closed vessel is preferably an ampoule (20) with a sterile interior.

5. The closed vessel according to claim 4, comprising end caps (22) on one side or on both sides, a membrane (24) and a plunger (25) which is slidingly sealed (26), wherein the plunger (25) has a central recess.

6. An injection device (40), comprising

   - a cannula (43), mounted on the front side of the injection device (40), the end of which, projecting into the interior of the injection device (40), has cutting edges,
   - a screw cap (42), placed on the rear side of the injection device, which comprises a central threaded hole,
   - a threaded pin (41) which is guided through the central threaded hole of the screw cap (42),
   - a manual rotary knob (45) which is arranged on the threaded pin (41),

   wherein between the front side and the rear side of the injection device (40) a cavity is formed which receives a closed vessel according to any one of claims 4 or 5.

7. The injection device (40) according to claim 6, **characterized in that** it is configured in order to measure the injection pressure on injecting a PVA gel through the cannula (43).

8. A method for the application of a PVA gel gelling in situ, **characterized in that** the method comprises the following steps:

   a) melting of the gel (1)
   b) optionally waiting (2)
   c) cooling of the gel (3)
   d) injection of the gel (4)
   e) gelation (5)

9. The method according to claim 8, **characterized in that** the PVA gel is a PVA gel according to any one of claims 1 to 3.

10. The method according to claim 9, **characterized in that** the PVA gel in the steps a) and b) is situated in a closed vessel according to any one of claims 4 or 5, which is opened during or after step c).

11. The method according to claim 10, **characterized in that** it is carried out by means of an injection device according to any one of claims 6 or 7.

12. The method according to any one of claims 8 to 11, **characterized in that**

   - the PVA gel in step a) is melted at a temperature > 85 °C,
   - the PVA gel in step c) is cooled to a temperature in a range of 30 to 80 °C and the cooling time is in particular < 30 min, and

- the PVA gel in step d) is injected at a temperature in a range of 30 to 80 °C.

13. The method according to any one of claims 8 to 12, **characterized in that** during the injection in step d) a pressure of < 20 MPa is reached.

14. The method according to any one of claims 8 to 13, **characterized in that** the injection in step d) takes place by means of a cannula and the cannula for the injection of the PVA gel is introduced into the annulus by means of a dilatation method.

15. Use of the PVA gel according to any one of claims 1 to 3 in biomedicine, in particular in orthopaedics, preferably in intervertebral discs concerning nucleus replacement.

**Revendications**

1. Gel d'alcool polyvinylique (PVA) à gélification in situ, **caractérisé en ce que**

   a) le gel de PVA comporte du PVA (composant i) et de l'eau (composant ii) en tant qu'agent de gonflage,
   b) la viscosité (viscosité dynamique) dudit gel en fusion est comprise entre 60 et 2000 Pas lorsque la température est comprise entre 30 et 100 °C,
   c) la teneur en eau W dudit gel de PVA est comprise entre 52 et 64 % en poids,
   d) pour ledit PVA, la limite supérieure du degré de polymérisation moyen en poids Pw est donnée par la relation Pw = W·150 - 5500, et
   e) pour ledit PVA, la limite inférieure du degré de polymérisation moyen en poids Pw est donnée par la relation Pw = W·90 - 3800.

2. Gel de PVA à gélification in situ selon l'une des revendications précédentes, **caractérisé en ce que** ledit gel de PVA comporte au moins deux types de PVA, à savoir le PVA1 et le PVA2, ledit PVA1 présentant un degré de polymérisation moyen en poids > 1500 et le PVA2 < 1500, et la proportion de PVA1 en % poids étant comprise entre 5 et 60 % en poids, par rapport à la quantité totale de PVA1 et de PVA2.

3. Gel de PVA à gélification in situ selon l'une des revendications précédentes 1 ou 2, **caractérisé en ce que** ledit gel de PVA comporte au moins une charge aux propriétés d'agent de contraste radiographique.

4. Récipient hermétique, contenant ledit Gel de PVA à gélification in situ selon l'une des revendications précédentes 1 à 3, ledit récipient hermétique étant de préférence une ampoule (20) dont l'intérieur est stérile.

5. Récipient hermétique selon la revendication 4, comprenant des capuchons (22) sur l'une de ses extrémités ou sur ses deux extrémités, une membrane (24) et un poinçon (25) pouvant glisser sans rompre l'étanchéité (26), ledit poinçon (25) étant pourvu d'un creux central.

6. Dispositif d'injection (40), comprenant

   - une canule (43), montée sur la face avant dudit dispositif d'injection (40), dont l'extrémité se prolongeant dans l'intérieur dudit dispositif d'injection (40) est pourvue de bords coupants,
   - un capuchon à pas de vis (42) qui est placé sur la face arrière dudit dispositif d'injection pourvue d'un trou taraudé central,
   - une tige filetée (41) passant à travers ledit trou taraudé central dudit capuchon à pas de vis (42),
   - un bouton rotatif à action manuelle (45), disposé sur ladite tige filetée (41),

   une enceinte étant formée entre la face avant et la face arrière dudit dispositif d'injection (40), laquelle sert de logement à un récipient hermétique selon l'une des revendications 4 ou 5.

7. Dispositif d'injection (40) selon la revendication 6, **caractérisé en ce qu'**il est disposé de façon à ce qu'il mesure la pression d'injection lors qu'un gel de PVA est injecté à travers ladite canule (43).

8. Procédé d'application d'un gel de PVA à gélification in situ, **caractérisé en ce que** ledit procédé comporte les étapes suivantes :

a) la mise en fusion dudit gel (1)
b) le cas échéant, le respect d'un délai d'attente (2)
c) le refroidissement dudit gel (3)
d) l'injection dudit gel (4)
e) la gélification (5)

**9.** Procédé selon la revendication 8, **caractérisé en ce que** ledit gel de PVA est un gel de PVA selon l'une des revendications 1 à 3.

**10.** Procédé selon la revendication 9, **caractérisé en ce que** ledit gel de PVA se trouve, aux étapes a) et b), dans un récipient hermétique selon l'une des revendications 4 ou 5 qui sera ouvert pendant ou après l'étape c).

**11.** Procédé selon la revendication 10, **caractérisé en ce qu'**il est mis en ouvre au moyen d'un dispositif d'injection selon l'une des revendications 6 ou 7.

**12.** Procédé selon l'une des revendications 8 à 11, **caractérisé en ce que**

- ledit gel de PVA est mis en fusion, à l'étape a), à une température > 85 °C,
- ledit gel de PVA est refroidi, à l'étape c), à une température comprise entre 30 et 80 °C et le délai de refroidissement est notamment < 30 min, et
- ledit gel de PVA est injecté, à l'étape d), à une température comprise entre 30 et 80 °C.

**13.** Procédé selon l'une des revendications 8 à 12, **caractérisé en ce que**, pendant ladite injection à l'étape d), la pression atteint une valeur < 20 MPa.

**14.** Procédé selon l'une des revendications 8 à 13, **caractérisé en ce que** ladite injection à l'étape d) est réalisée au moyen d'une canule et ladite canule est introduite dans l'annulus au moyen d'un procédé de dilatation pour ainsi injecter ledit gel de PVA.

**15.** Utilisation dudit gel de PVA selon l'une des revendications 1 à 3 dans la biomédecine, notamment dans l'orthopédie, de préférence au niveau des disques intervertébraux lors d'un remplacement du nucleus.

**Figur 1**

**Figur 2**

**Figur 3**

**Figur 4**

**Figur 5**

**Figur 6**

**Figur 7**

**Figur 8**

**Figur 9**

**Figur 10**

**Figur 11**

**Figur 12**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2005017000 A1
- WO 2007050744 A
- US 7214245 B1
- WO 9210982 A1
- WO 2006021122 A2
- WO 2006021122 A

**In der Beschreibung aufgeführte Nicht-Patentliteratur**

- **T. NOGUCHI ; T. YAMAMURO et al.** *Journal of Applied Biomaterials,* 1991, vol. 2, 101-107
- **R. A. BADER ; W. E. ROCHEFORT.** *Journal of Biochemical Materials Research Part A,* 2007, vol. 86a (2), 494-501
- **J. THOMAS et al.** *Journal of Biomedical Research Part A,* 2003, vol. 67a (4), 1329-1337